# EUROPÄISCHE PATENTSCHRIFT

(11) **EP 1 491 617 B1**
(45) Veröffentlichungstag und Bekanntmachung des Hinweises auf die Patenterteilung: **27.09.2006**
(21) Anmeldenummer: 03014144.4
(22) Anmeldetag: 24.06.2003
(51) Int. Cl.: C11D 1/62, C11D 3/00

(54) **Perlglänzende wässrige Zubereitungen**
Aqueous pearlescent compositions
Compositions aqueuses nacrées

(43) Veröffentlichungstag der Anmeldung: 29.12.2004
(73) Patentinhaber: Cognis IP Management GmbH, 40589 Düsseldorf (DE)
(72) Erfinder: Bigorra Llosas, Joaquim, Dr., 08203 Sabadell (ES); Bonastre Gilabert, Nuria, Dr., 08210 Barbera del Vall (ES); Sanchez, Agustin, 08921 Santa Coloma De Gramenet (ES)

(56) Entgegenhaltungen:
- WO-A-98/52907
- WO-A-99/06514
- US-A- 5 437 801

## Beschreibung

### Gebiet der Erfindung

Die Erfindung befindet sich auf dem Gebiet der kationischen Tenside und betrifft neue wässrige Zubereitungen, die sich durch einen perlglänzenden Effekt auszeichnen.

### Stand der Technik

Quaternierte Fettsäuretriethanolaminestersalze, die zur Gruppe der Esterquats zählen, stellen kationische Tenside dar, die leicht auf synthetische wie natürliche Fasern aufziehen und diesen Substantivität und einen angenehmen Weichgriff verleihen. Wässrige Dispersionen dieser Stoffe werden daher als Avivagemittel sowohl im Bereich der Textilpflege als auch für die Konditionierung von Haaren eingesetzt. Zu ihrer Herstellung geht man im allgemeinen von Fettsäuren aus, die man mit Triethanolamin in Gegenwart von unterphosphoriger Säure mit partiell verestert und anschließend in einer Lösung von niederen Alkoholen oder Polyolen mit Alkylhalogeniden oder Dialkylsulfaten, vorzugsweise Methylchlorid oder Dimethylsulfat quaterniert. In der Literatur wird dabei in der Regel ein molaren Einsatzverhältnis von Triethanolamin zu Fettsäure von 1 : 1,2 bis 1 : 2,2 beschrieben, wobei aus anwendungstechnischen Gründen vorzugsweise ein Verhältnis von 1 : 1,5 bis 1 : 1,9 realisiert wird.

Aus ästhetischen Gründen besteht am Markt ein Interesse daran, Formulierungen dieser Esterquats nicht nur in transparenter Form anzubieten, sondern insbesondere auch perlglänzende Mittel zur Verfügung zu stellen. Zu diesem Zweck hat es nicht an Versuchen gefehlt, den Dispersionen entsprechende Wachskörper zuzusetzen, jedoch ohne durchschlagenden Erfolg, da sich die Wachse nicht über längere Zeit stabil in die Formulierung einarbeiten lassen.

Somit hat die Aufgabe der vorliegenden Erfindung darin bestanden, neue wässrige Zubereitungen auf Basis von quaternierten Fettsäuretriethanolaminestersalzen zur Verfügung zu stellen, die ohne Zusatz von Wachskörpern über perlglänzende Eigenschaften verfügen und dabei im Vergleich zu den Produkten des Stands der Technik über gleichwertige anwendungstechnische Eigenschaften verfügen.

### Beschreibung der Erfindung

Gegenstand der Erfindung sind perlglänzende wässrige Zubereitungen, die dadurch erhältlich sind, dass man
(a) Triethanolamin und Fettsäuren mit 12 bis 22 Kohlenstoffatomen im molaren Verhältnis 1 : 1,32 bis 1 : 1,38 : umsetzt,
(b) die Veresterungsprodukte anschließend in an sich bekannter Weise quaterniert und
(c) die Quaternierungsprodukte in Wasser dispergiert.

Überraschenderweise wurde gefunden, dass sich über die Steuerung des Veresterungsgrades und der Fettsäure Esterquats herstellen lassen, die in wässriger Dispersion selbst perlglänzende Eigenschaften besitzen, die Mitverwendung üblicher Perlglanzwachse, wie z.B. vom Typ der Glycolfettsäureester nicht erforderlich ist. Die Erfindung schließt die Erkenntnis ein, dass das molare Einsatzverhältnis zwischen Triethanolamin und den ausgewählten Fettsäuren den kritischen Parameter darstellt: ein zu niedriges Verhältnis führt zu geringem Perlglanz und insbesondere ungenügenden anwendungstechnischen Eigenschaften, bei einem zu hohen Verhältnis wird kein Perlglanzeffekt mehr beobachtet.

Ein weiterer Gegenstand der Erfindung betrifft ein Verfahren zur Herstellung perlglänzender wässriger Zubereitungen, bei dem man
(a) Triethanolamin und Fettsäuren mit 12 bis 22 Kohlenstoffatomen im molaren Verhältnis 1 : 1,32 bis 1 : 1,38 umsetzt,
(b) die Veresterungsprodukte anschließend in an sich bekannter Weise quaterniert und
(c) die Quaternierungsprodukte in Wasser dispergiert.

Vorzugsweise enthalten die Zubereitungen als Konsequenz aus dem unterstöchiometrischen Einsatz der Fettsäuren - bezogen auf den Trockenrückstandes - einen Gehalt an freien Fettsäuren mit 12 bis 22 Kohlenstoffatomen von 1 bis 20, vorzugsweise 3 bis 15 und insbesondere 5 bis 10 Gew.-%.

### Einsatzstoffe

Die Auswahl der Einsatzstoffe ist hinsichtlich der Alkanolaminkomponente auf Triethanolamin beschränkt, da beispielsweise mit Methyldiethanolamin deutlich weniger befriedigende Ergebnisse erzielt werden. Bezüglich der Auswahl der Fettsäurekomponente kommen vorzugsweise solche in Frage, die der Formel **(I)** folgen,

**R**^{**1**}**COOH** **(I)**

in der R¹CO für einen linearen oder verzweigten, gesättigten oder ungesättigten Acylrest mit 16 bis 18 Kohlenstoffatomen steht. Insbesondere hat sich der Einsatz von gehärteteter oder teilgehärteter Kokos- oder Talgfettsäure, sowie vorzugsweise von Palmitin-, Stearin- und Behensäure sowie deren Gemischen bewährt.

### Veresterung und Quaternierung

Zur Herstellung der Esterquats kann sowohl von Fettsäuren als auch den entsprechenden Triglyceriden ausgegangen werden. Ebenfalls ist es möglich, die Kondensation der Alkanolamine mit den Fettsäuren in Gegenwart definierter Mengen an Dicarbonsäuren, wie z.B. Oxalsäure, Malonsäure, Bernsteinsäure, Maleinsäure, Fumarsäure, Glutarsäure, Adipinsäure, Sorbinsäure, Pimelinsäure, Azelainsäure, Sebacinsäure und/oder Dodecandisäure durchzuführen. Auf diese Weise kommt es zur einer partiell oligomeren Struktur der Esterquats. Im Hinblick auf eine besondere Ausgewogenheit von Perlglanzeffekt und anwendungstechnischer Leistung, insbesondere Weichgriff, hat es sich als optimal erwiesen, Triethanolamin und Fettsäuren im molaren Verhältnis von 1 : 1,32 bis 1 : 1,38 einzusetzen. Für die Quaternierung eignen sich Alkylhalogenide oder Dialkylsulfate, insbesondere Methylchlorid oder Dimethylsulfat. Sowohl die Veresterung als auch die Quaternierung kann in an sich bekannter Weise durchgeführt werden. Stellvertretend für den umfangreichen Stand der Technik sei auf die beiden Druckschriften **DE 4308794 C1** und **DE 4335782 C1** (Cognis) verwiesen, deren Lehre von der vorliegenden Patentanmeldung miteingeschlossen wird. Insbesondere erfolgt die Herstellung der erfindungsgemäßen quaternierten Fettsäuretriethanolaminestersalze in Gegenwart niederer aliphatischer Alkohole oder Polyole, die den Zwischenprodukten vor der Quaternierung zugesetzt werden. Für diesen Zweck eignen sich C₁-C₄ Alkohole sowie Alkylenglycole mit 2 bis 10 Kohlenstoffatomen, insbesondere aber Isopropylalkohol oder Propylenglycol. Die Herstellung solcher Produkte wird beispielsweise in der **DE 19738645 C1** (Cognis) beschrieben. Aus der Quaternierung werden in der Regel 60 bis 95, vorzugsweise 75 bis 85 Gew.-%ige Zubereitungen der quaternierten Fettsäuretriethanolaminestersalze in den niederen Alkoholen bzw. Polyolen erhalten, die dann mit Wasser auf eine Feststoffkonzentration von 5 bis 40, vorzugsweise 10 bis 30 Gew.-% verdünnt werden und innerhalb dieses Konzentrationsbereiches auch einen perlglanz aufweisen.

### Gewerbliche Anwendbarkeit

Ein weiterer Gegenstand der vorliegenden Erfindung betrifft die Verwendung der neuen perlglänzenden wässrigen Zubereitungen zur Herstellung von Avivagemitteln für synthetische und/oder natürliche Fasern, in denen sie in Mengen von 10 bis 100 Gew.-% - bezogen auf die Endzubereitung - enthalten sein können. Im einfachsten Fall können die Zubereitungen selbst als Avivagemittel eingesetzt werden, es ist jedoch auch möglich sie mit Wasser weiter zu verdünnen und/oder andere übliche Hilfs- und Zusatzstoffe hinzuzufügen. Letzteres ist insbesondere dann der Fall, wenn die Zubereitungen als Conditioner im Bereich der Haarkosmetik eingesetzt werden sollen. Bezüglich der in Frage kommenden Zusatzstoffe sei wieder auf die bereits erwähnte **DE 19738645 C1** verwiesen.

### Beispiele

### Allgemeine Herstellvorschrift

In einem 1,5-1-Dreihalskolben mit Rührer, Tropftrichter und Destillationsaufsatz wurden 596 bis 943 g (2,88 bis 4,56 Mol) Stearinsäure (StA) und 1,2 bis 1,8 g Hypophosphorsäure (50 Gew.-%ig) vorgelegt und auf 70°C erhitzt. Bei einem verminderten Druck von 30 mbar wurden portionsweise 363 g (2,4 Mol) Triethanolamin (TEA) zugetropft und dabei die Temperatur bis auf 160°C gesteigert. Nach dem Ende der Zugabe wurde die Reaktionsmischung noch weitere 2 h bei 2 mbar gerührt, bis kein weiteres Reaktionswasser abgeschieden wurde und die Säurezahl einen Wert unterhalb von 5 mg KOH/g erreicht hatte. Anschließend wurden 400 g (0,86 Mol) des hergestellten Esters in einen zweiten Dreihalskolben überführt und bei 50°C in 126 g Propylenglycol gelöst. Anschließend wurden portionsweise 104 g (0,83 Mol) Dimethylsulfat zugetropft und die Mischung 4 h bei 65°C gerührt. Es wurde eine bei 20°C niedrigviskose Lösung erhalten, die 80 Gew.-% Esterquat und 20 Gew.-% Propylenglycol enthielt. Die Lösung wurde anschließend mit einer solchen Menge Wasser versetzt, dass eine Dispersion mit einem Feststoffgehalt von 15 Gew.-% erhalten wurde.

Die perlglänzenden Eigenschaften der Dispersionen wurden subjektiv auf einer Skala von 1 (= brillanter Perlglanz) bis 4 (= kein Effekt vorhanden) bewertet. Zur Bestimmung der anwendungstechnischen Eigenschaften wurde 1 kg Frotteewäsche in einer haushaltsüblichen Waschmaschine des Typs Miele bei 60 °C gewaschen und der Flotte dabei eine Messkappe der Weichspülerdispersionen zugesetzt. Nach dem Trocknen wurde der Weichgriff von einem Panel bestehend aus 5 erfahrenen Testern auf einer Skala von 1 (= sehr weich) bis 4 (= hart) bestimmt und dann gemittelt. Die Ergebnisse sind in Tabelle 1 zusammengefasst. Die Beispiele 1 bis 3 sind erfindungsgemäß, die Beispiele V1 bis V3 dienen zum Vergleich.

**Tabelle 1**

| **Perlglanzeffekt und Weichgriff** | | | | | |
|---|---|---|---|---|---|
| Bsp. | TEA (g) | StA (g) | Molares Verhältnis TEA : KFS | Perlglanz | Weichgriff |
| V1 | 363 | 596 | 1:1,20 | 2 | 4 |
| 1* | 363 | 645 | 1:1,30 | 1 | 3 |
| 2 | 363 | 671 | 1 : 1,35 | 1 | 2 |
| 3* | 363 | 695 | 1 : 1,40 | 1 | 2 |
| V2 | 363 | 745 | 1 : 1,50 | 3 | 2 |
| V3 | 363 | 795 | 1 : 1,60 | 4 | 1 |
| V4 | 363 | 943 | 1 : 1,90 | 4 | 1 |

| | | | | | |
|---|---|---|---|---|---|
| * Beispiel außerhalb des Schutzbereichs der Ansprüche | | | | | |

Man erkennt, dass bei einem Einsatzverhältnis unterhalb des erfindungsgemäßen Bereiches von TEA:KFS = 1 : 1,3 zwar noch ein akzeptabler Perlglanz, jedoch kein Weichgriff mehr erzielt wird, während oberhalb der erfindungsgemäßen Grenze von 1 : 1,4 die anwendungstechnischen Eigenschaften zwar sehr gut sind, jedoch kein Perlglanzeffekt mehr besteht.

## Patentansprüche

1. Perlglänzende wässrige Zubereitungen, dadurch erhältlich, dass man
(a) Triethanolamin und Fettsäuren mit 12 bis 22 Kohlenstoffatomen im molaren Verhältnis 1 : 1,32 bis 1 : 1,38 umsetzt,
(b) die Veresterungsprodukte anschließend in an sich bekannter Weise quaterniert und
(c) die Quaternierungsprodukte in Wasser dispergiert.

2. Zubereitungen nach Anspruch 1, **dadurch gekennzeichnet, dass** sie - bezogen auf den Trockenrückstand - einen Gehalt an freien Fettsäuren mit 12 bis 22 Kohlenstoffatomen von 1 bis 20 Gew.-% aufweisen.

3. Verfahren zur Herstellung perlglänzender wässriger Zubereitungen, bei dem man
(a) Triethanolamin und Fettsäuren mit 12 bis 22 Kohlenstoffatomen im molaren Verhältnis 1 : 1,32 bis 1 : 1,38 umsetzt,
(b) die Veresterungsprodukte anschließend in an sich bekannter Weise quaterniert und
(c) die Quaternierungsprodukte in Wasser dispergiert.

4. Verfahren nach Anspruch 3, **dadurch gekennzeichnet, dass** man Fettsäuren der Formel (I) einsetzt,
**R**^{**1**}**COOH** **(I)**
in der R¹CO für einen linearen oder verzweigten, gesättigten oder ungesättigten Acylrest mit 16 bis 18 Kohlenstoffatomen steht.

5. Verfahren nach Anspruch 4, **dadurch gekennzeichnet, dass** man gehärtetete oder teilgehärtete Kokos- oder Talgfettsäure, Palmitinsäure oder Stearinsäure sowie deren Gemische einsetzt.

6. Verfahren nach mindestens einem der Ansprüche 3 bis 5, **dadurch gekennzeichnet, dass** man die Veresterungsprodukte mit Alkylhalogeniden oder Dialkylsulfaten quaterniert.

7. Verfahren nach mindestens einem der Ansprüche 3 bis 6, **dadurch gekennzeichnet, dass** man die Alkylierung in Gegenwart niederer aliphatischer Alkohole oder Polyole durchführt.

8. Verfahren nach Anspruch 7, **dadurch gekennzeichnet, dass** man die Quaternierung in Gegenwart von Isopropylalkohol oder Propylenglycol durchführt.

9. Verfahren nach mindestens einem der Ansprüche 3 bis 8, **dadurch gekennzeichnet, dass** man 60 bis 95 Gew.-%ige Zubereitungen der quaternierten Fettsäuretriethanolaminestersalze in den niederen Alkoholen bzw. Polyolen herstellt und diese mit Wasser auf eine Feststoffkonzentration von 5 bis 40 Gew.-% verdünnt.

10. Verwendung von perlglänzenden wässrigen Zubereitungen gemäß Anspruch 1 zur Herstellung von Avivagemitteln für synthetische und/oder natürliche Fasern.

## Claims

1. Pearlescent water-containing preparations which can be obtained by
(a) reacting triethanolamine and C₁₂₋₂₂ fatty acids in a molar ratio of 1:1.32 to 1:1.38,
(b) subsequently quaternizing the esterification products in known manner and
(c) dispersing the quaternization products in water.

2. Preparations as claimed in claim 1, **characterized in that** they have a content of free C₁₂₋₂₂ fatty acids of 1 to 20% by weight, based on the dry residue.

3. A process for the production of pearlescent water-containing preparations, in which
(a) triethanolamine and C₁₂₋₂₂ fatty acids are reacted in a molar ratio of 1:1.32 to 1:1.38,
(b) the esterification products are then quaternized in known manner and
(c) the quaternization products are dispersed in water.

4. A process as claimed in claim 3, **characterized in that** fatty acids corresponding to formula (I):
**R**^{**1**}**COOH** **(I)**
in which R¹CO is a linear or branched, saturated or unsaturated acyl group containing 16 to 18 carbon atoms.
are used.

5. A process as claimed in claim 4, **characterized in that** hydrogenated or partly hydrogenated coconut oil or tallow fatty acid, palmitic acid or stearic acid or mixtures thereof are used.

6. A process as claimed in at least one of claims 3 to 5, **characterized in that** the esterification products are quaternized with alkyl halides or dialkyl sulfates.

7. A process as claimed in at least one of claims 3 to 6, **characterized in that** the alkylation is carried out in the presence of lower aliphatic alcohols or polyols.

8. A process as claimed in claim 7, **characterized in that** the quaternization is carried out in the presence of isopropyl alcohol or propylene glycol.

9. A process as claimed in at least one of claims 3 to 8, **characterized in that** 60 to 95% by weight preparations of the quaternized fatty acid triethanolamine ester salts in the lower alcohols or polyols are prepared and are diluted with water to a solids concentration of 5 to 40% by weight.

10. The use of the pearlescent water-containing preparations claimed in claim 1 for producing softeners for synthetic and/or natural fibres.

## Revendications

1. Préparations aqueuses à éclat nacré, que l'on peut obtenir :
(a) en faisant réagir de la triéthanolamine et des acides gras ayant 12 à 22 atomes de carbone dans un rapport molaire de 1/1,32 à 1 / 1,38,
(b) en quaternisant ensuite les produits d'estérification d'une manière connue en elle même, et
(c) en dispersant les produits de quaternisation dans l'eau.

2. Préparations selon la revendication 1,
**caractérisées en ce qu'**
elles présentent (par rapport au résidu sec) une teneur en acides gras libres ayant 12 à 22 atomes de carbone de 1 à 20 % en poids.

3. Procédé d'obtention de préparations aqueuses à éclat nacré, consistant à
(a) faire réagir de la triéthanolamine et des acides gras ayant 12 à 22 atomes de carbone dans un rapport molaire de 1/1,32 à 1 / 1,38,
(b) quaterniser ensuite les produits d'esterification d'une manière connue en elle même, et
(c) disperser les produits de quaternisation dans l'eau.

4. Procédé selon la revendication 3,
**caractérisé en ce qu'**
on utilise des acides gras répondant à la formule (I)
R¹ COOH (I)
dans laquelle R¹CO représente un radical acyle, linéaire ou ramifié, saturé ou insaturé, ayant 16 à 18 atomes de carbone.

5. Procédé selon la revendication 4,
**caractérisé en ce qu'**
on utilise l'acide gras de coprah ou de graisse de suif durci ou partiellement durci, l'acide palmitique ou l'acide stéarique, ainsi que leurs mélanges.

6. Procédé selon au moins l'une des revendications 3 à 5,
**caractérisé en ce qu'**
on quaternise les produits d'estérification avec des halogénures d'alkyle ou des sulfates de dialkyle.

7. Procédé selon au moins l'une des revendications 3 à 6,
**caractérisé en ce qu'**
on effectue l'alkylation en présence d'alcools ou de polyols aliphatiques inférieurs.

8. Procédé selon la revendication 7,
**caractérisé en ce qu'**
on effectue la quaternisation en présence d'alcool isopropylique ou de propylèneglycol.

9. Procédé selon au moins l'une des revendications 3 à 8,
**caractérisé en ce qu'**
on produit des préparations à 60 à 95 % en poids de sels d'esters de triéthanolamine d'acides gras quaternisés dans les alcools ou polyols inférieurs et on les dilue avec de l'eau jusqu'à une concentration de matière solide de 5 à 40 % en poids.

10. Utilisation de préparations aqueuses à éclat nacré selon la revendication 1, pour l'obtention d'agents d'avivage pour fibres synthétiques et/ou naturelles.
